(19) Europäisches Patentamt
European Patent Office
Office européen des brevets

(11) Veröffentlichungsnummer: **0 514 790 A2**

(12) # EUROPÄISCHE PATENTANMELDUNG

(21) Anmeldenummer: **92108251.7**

(22) Anmeldetag: **15.05.92**

(51) Int. Cl.5: **C08G 63/16**, C08G 69/10, C08G 75/26, C08G 69/26, A61K 9/22, A61K 9/16, A61K 9/52, A61K 47/32, A61K 49/00

(30) Priorität: **24.05.91 DE 4116936**

(43) Veröffentlichungstag der Anmeldung:
**25.11.92 Patentblatt 92/48**

(84) Benannte Vertragsstaaten:
**AT BE CH DE DK ES FR GB GR IT LI LU NL PT SE**

(71) Anmelder: **HOECHST AKTIENGESELLSCHAFT**
**Postfach 80 03 20**
**W-6230 Frankfurt am Main 80(DE)**

(72) Erfinder: **Krone, Volker, Dr.**
**Zu den Eichen 25**
**W-6238 Hofheim am Taunus(DE)**
Erfinder: **Magerstädt, Michael, Dr.**
**Theodor-Heuss-Strasse 14**
**W-6073 Egelsbach(DE)**
Erfinder: **Walch, Axel, Dr.**
**Hans-Sachs-Strasse 5**
**W-6000 Frankfurt am Main(DE)**
Erfinder: **Ditzinger, Günter, Dr.**
**Storchgasse 23**
**W-6230 Frankfurt am Main 80(DE)**
Erfinder: **Lill, Norbert, Dr.**
**Fichtenstrasse 42a**
**W-6242 Kronberg/Ts.(DE)**

(54) **Polykondensate, die Weinsäurederivate enthalten, Verfahren zu ihrer Herstellung und Verwendung derselben.**

(57) Die Erfindung betrifft Polykondensate, die aus mindestens 95 Mol-% an wiederkehrenden Struktureinheiten der Formel I bestehen,

$$[-\overset{O}{\overset{\|}{C}}-R^1-\overset{O}{\overset{\|}{C}}-X-R^2-X-] \qquad (I)$$

wobei $R^1$ für 2,3-O-Alkylidenweinsäurederivat (II) oder Furo [2,5] (V), Alkyl, Alkenyl, Cycloalkyl, Cycloalkenyl, Aryl oder Heterocyclus steht, X für -O-, -NH- oder - S- und $R^2$ für 2,3-O-Alkyliden-L-threitol (III) oder Verbindung der Formel VI, Alkyl, Alkenyl, Cycloalkyl, Cycloalkenyl, Aryl oder Heterocyclus steht mit der Maßgabe, daß mehr als 5 Mol-% der Reste $R^1$ und/oder $R^2$ in dem Polykondensat der Formel I enthalten sind, wobei $R^1$ für eine Verbindung der Formel II oder $R^2$ für eine Verbindung der Formel III oder $R^1$ für eine Verbindung der Formel II und $R^2$ für eine Verbindung der Formel III steht, oder daß mehr als 5 Mol-% der Reste $R^1$ und $R^2$ in dem Polykondensat der Formel I enthalten sind, wobei

a) $R^1$ für eine Verbindung der Formel V und $R^2$ für eine Verbindung der Formel VI steht, oder

b) $R^1$ für Phenyl[1,2], Phenyl[1,3] oder Phenyl[1,4] und $R^2$ für eine Verbindung der Formel VI steht,

Verfahren zur Herstellung dieser Polykondensate, sowie deren Verwendung zur Herstellung von Depotzubereitungen mit kontrollierter Wirkstoffabgabe oder zur Herstellung von Ultraschall-Kontrastmitteln.

EP 0 514 790 A2

Die Erfindung betrifft Polykondensate, die durch die Anwesenheit von Weinsäurederivaten gekennzeichnet sind, Verfahren zu ihrer Herstellung und Verwendung derselben für Depotzubereitungen mit kontrollierter Wirkstoffabgabe oder zur Herstellung von Ultraschall-Kontrastmitteln.

Eine moderne Arzneitherapie erfordert insbesondere zur Applikation von Wirkstoffen neue Darreichungsformen, die eine kontrollierte Abgaberate der Wirkstoffe mit hoher Biokompatibilität des Depots vereinigen. Eine langandauernde kontrollierte Wirkstoffabgabe ist wegen der zunehmenden Bedeutung chronischer Erkrankungen und langzeitorientierter Therapiekonzepte in Human- und Veterinärmedizin von großer Aktualität. Als Matrixmaterialien für solche Depotsysteme sind bioabbaubare Polymere besonders vorteilhaft, da die Bioerosion im wesentlichen die Wirkstofffreisetzung steuert und die chirurgische Entfernung eines derartigen Depots entbehrlich macht.

In biologisch abbaubaren Arzneistoffabgabesystemen, wie in dem amerikanischen Patent 4,093,709 angegeben, wird der Wirkstoff in einem bioabbaubaren Polymeren dispergiert, welches beim Abbau den Wirkstoff freigibt. Typische, nach dem Stand der Technik meist untersuchte, biologisch abbaubare Polymere sind Homo- und Copolyester, insbesondere der Milch- und Glykolsäure, wie sie in den US-Patenten 3,773,919 bzw. 3,297,033 beschrieben sind. Nachteilig sind u. a. die schlecht kontrollierbare Quellbarkeit der Polyester im physiologischen Milieu und der damit verbundene komplexe Mechanismus der Wirkstofffreisetzung. Zusätzlich wird im allgemeinen nach einem erheblichen "initial burst" nur eine geringe bis mäßige Freisetzungsrate bewirkt.

Nicht nur in der Therapie, auch in der Diagnostik bewähren sich Polymere enthaltende Präparate immer häufiger. So z. B. in der Ultraschalldiagnostik. Sie hat in der Medizin wegen der komplikationslosen einfachen Handhabung sehr breite Anwendung gefunden. Ultraschallwellen werden an Grenzflächen von unterschiedlichen Gewebearten reflektiert. Die dabei entstehenden Echosignale werden elektronisch verstärkt und sichtbar gemacht.

Die Darstellung von Blutgefäßen und inneren Organen mittels Ultraschall erlaubt im allgemeinen nicht die Darstellung des darin vorhandenen Blutflusses. Flüssigkeiten, insbesondere Blut, liefern nur dann Ultraschallkontrast, wenn Dichteunterschiede zur Umgebung bestehen. Als Kontrastmittel werden in der medizinischen Ultraschalldiagnostik z. B. Gase enthaltende oder Gase produzierende Substanzen verwendet, da der Impedanzunterschied zwischen Gas und umgebendem Blut wesentlich größer ist, als der zwischen Flüssigkeiten oder Festkörpern und Blut (Levine R. A., J Am Coll Cardiol 3: 28, 1989; Machi I. J CU 11: 3, 1983).

In der EP-A1-0,327,490 werden Mikropartikel beschrieben die aus Amylosen oder synthetischen, bioabbaubaren Polymeren und einem Gas und/oder einer Flüssigkeit mit einem Siedepunkt von weniger als 60 °C, bestehen. Nachteile dieser Polymere sind deren klebrige Konsistenz in Wasser oder Blut, deren schlechte Bioabbaubarkeit oder der Möglichkeit das toxische Abbauprodukte entstehen können.

Bisher wurde die Herstellung von einigen sehr niedermolekularen, geschützten Weinsäureestern beschrieben (Schacht et al. Makromol Chem. 179, (1978), 837 - 840), die eine Viskosität von 0,04 $dl \cdot g^{-1}$, gemessen in Dimethylformamid bei 25 °C, zeigen. Die beschriebenen Viskositäten zeigen, daß es sich nicht um Polymere der geschützten Weinsäurederivate handelt.

Aufgabe der vorliegenden Erfindung ist es, Polykondensate zu finden, die eine mechanisch stabile Struktur aufweisen, die gut suspendierbar in Wasser sind, ein geringes Quellungsverhalten in Wasser aufweisen und keine schmierig klebrige Konsistenz haben, die leicht zu weitgehend untoxischen Produkten chemisch oder biologisch abbaubar sind und deren Abbauprodukte wasserlöslich sind.

Es wurde nun gefunden, daß Polykondensate die 2,3-0-Alkylidenweinsäurederivate,2,3-0-Alkyliden-L-threitol, Furo[2,5]-gruppen oder Terepthalate enthalten, die gewünschten Eigenschaften aufweisen.

Überraschenderweise zeigten die Polykondensate, wenn sie für Depotzubereitungen von Arzneimitteln verwendet werden, eine gleichmäßige kontrollierbare Wirkstoffabgabe mit stark vermindertem "initial burst". Ferner ließen sich nahezu farblos lösliche Polykondensate aus den entsprechenden Säuredichloriden herstellen.

Die Erfindung betrifft daher Polykondensate, die im wesentlichen mindestens 95 Mol-% an wiederkehrenden Struktureinheiten der Formel I enthalten

$$[-\overset{\overset{O}{\|}}{C}-R^1-\overset{\overset{O}{\|}}{C}-X-R^2-X-] \qquad (I)$$

wobei $R^1$ für

a) Verbindung der Formel II

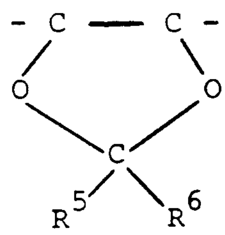

(II)

wobei $R^5$ und $R^6$ inerte Reste bedeuten,

b) geradkettiges oder verzweigtes Alkyl, Alkenyl, Cycloalkyl oder Cycloalkenyl, die mit einem oder mehreren inerten Resten substituiert sein können,

c) Aryl, ein- oder mehrkernig, ein- oder mehrfach substituiert durch inerte Reste,

d) Verbindungen der Formel V,

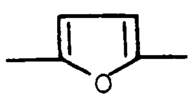

(V)

e) heterocyclischer Rest, der ein- oder mehrkernig und ein- oder mehrfach durch inerte Reste substituiert sein kann, steht

wobei X für

a) -O-,

b) -NH- oder

c) -S-

steht,

wobei $R^2$ für

a) Verbindung der Formel III,

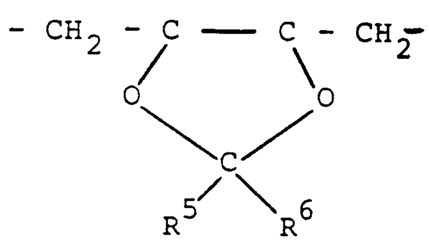

(III)

worin $R^5$ und $R^6$ die obengenannte Bedeutung haben,

b) geradkettiges oder verzweigtes Alkyl, Alkenyl, Cycloalkyl oder Cycloalkenyl, die mit einem oder mehreren inerten Resten substituiert sein können,

c) ein- oder mehrkerniges Aryl, ein- oder mehrfach substituiert durch inerte Reste,

d) heterocyclischer Rest, der ein- oder mehrkernig und ein- oder mehrfach durch inerte Reste substituiert sein kann, oder

e) Verbindung der Formel VI,

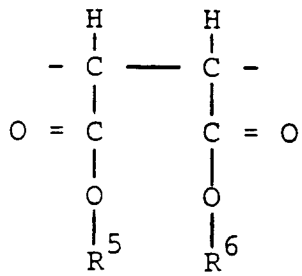

(VI)

worin $R^5$ und $R^6$ die obengenannte Bedeutung haben,

mit der Maßgabe, daß mehr als 5 Mol-% der Reste $R^1$ und/oder $R^2$ in dem Polykondensat der Formel I enthatten sind, wobei $R^1$ für eine Verbindung der Formel II und/oder $R^2$ für eine Verbindung der Formel III steht,

oder daß mehr als 5 Mol-% der Reste $R^1$ und $R^2$ in dem Polykondensat der Formel I enthalten sind, wobei

a) $R^1$ für eine Verbindung der Formel V und $R^2$ für eine Verbindung der Formel VI steht, oder

b) $R^1$ für Phenyl[1,2], Phenyl[1,3] oder Phenyl[1,4] und $R^2$ für eine Verbindung der Formel VI steht.

Unter dem Begriff "inerter Rest" werden Substituenten verstanden, die unter den Herstellungs- und Verarbeitungsbedingungen der erfindungsgemäßen Polykondensate nicht miteinander reagieren oder durch Schutzgruppen daran gehindert werden miteinander zu reagieren. Inerte Reste können beispielsweise anorganische Reste, wie Halogen sein, oder es kann sich um organische Reste, wie Alkyl, Alkenyl, Cycloalkyl, Cycloalkenyl, Alkoxy, oder Dialkylaminoalkyl handeln.

Funktionelle Gruppen, die durch Schutzgruppen an der Reaktion gehindert werden, sind beispielsweise Amino oder Hydroxy.

Bekannte Schutzgruppen sind z. B. die Benzyloxy- oder Phenylsulfonylgruppe.

Heterocyclische Reste können ein- oder mehrkernig sein und weisen insbesondere ein oder zwei Sauerstoff-, Stickstoff- oder Schwefelatome im Kern auf.

Aryl steht für einen aromatischen Kohlenstoffring. Mehrkernige Aryl-Reste können miteinander kondensiert, über C-C-Bindungen oder über Brückengruppen, wie z. B. - O-, -COO-, $-CH_2-$, -CO-NH-, -S-, -CO- oder eine $-SO_2$-Gruppe linear miteinander verbunden sein.

Beispiele für mehrkernige aromatische Aryl-Reste sind, 4,4'-Biphenyl, Naphthalin-1,5- oder -2,6-Gruppen oder Naphthalin-1,8-Gruppen.

Bevorzugt sind die Polykondensate der Formel I, die im wesentlichen mindestens 95 Mol-% an wiederkehrenden Struktureinheiten der Formel I enthalten, wobei $R^1$ für

a) geradkettig oder verzweigtes ($C_1$-$C_{18}$)-Alkyl oder geradkettig oder verzweigtes ($C_1$-$C_{18}$)-Alkenyl, das eine oder mehrere Doppelbindungen enthält,

b) geradkettig oder verzweigtes ($C_1$-$C_{18}$)-Alkyl oder geradkettig oder verzweigtes ($C_1$-$C_{18}$)-Alkenyl, das eine oder mehrere Doppelbindungen enthält, ein- oder mehrfach substituiert durch

1) ($C_5$-$C_7$)-Cycloalkyl,

2) ($C_5$-$C_7$)-Cycloalkenyl, mit einer oder mehrerer Doppelbindungen,

3) Halogen, wie Fluor oder Chlor,

4) Mercapto,

5) Hydroxy,

6) ($C_1$-$C_6$)-Alkoxy,

7) Phenoxy,

8) Phenoxy, ein- oder mehrfach substituiert durch

8.1 Halogen, wie Fluor, Chlor, Brom oder Jod,

8.2 ($C_1$-$C_6$)-Alkyl,

8.3 ($C_1$-$C_6$)-Alkoxy,

8.4 Nitro oder

8.5 Carbethoxy,

9) Naphthyloxy,

10) Benzyloxy,

11) Benzyloxy, ein- oder mehrfach substituiert im Arylteil durch

11.1 Methoxy,

11.2 Carboxyamido,

11.3 Amino,

11.4 Halogen, wie Fluor, Chlor, Brom oder Jod,

11.5 Nitro oder
11.6 Methyl,
12) Amino,
13) Monoalkylamino, mit bis zu 7 C-Atomen,
14) Monoalkylamino, mit bis zu 7 C-Atomen, ein- oder mehrfach substituiert im Alkylteil durch
14.1 Hydroxy,
14.2 Carboxy,
14.3 Carboxyamid,
14.4 Carboethoxy,
14.5 Amino,
14.6 ($C_1$-$C_6$)-Alkylamino,
14.7 Di-($C_1$-$C_6$)-alkylamino,
14.8 Piperidin oder
14.9 Morpholin,
15) Dialkylamino, mit bis zu 7 C-Atomen, oder
16) Dialkylamino, mit bis zu 7 C-Atomen, substituiert wie in b) 14.1 bis 14.9 definiert,

c) Aryl, mit 5 bis 14 Kohlenstoffatomen, gegebenenfalls ein- oder zweifach substituiert durch
1.1 ($C_1$-$C_6$)-Alkyl,
1.2 ($C_2$-$C_6$)-Alkenyl,
1.3 ($C_1$-$C_6$)-Alkylcarboxy,
1.4 Hydroxy,
1.5 Halogen, wie Fluor, Chlor, Brom oder Jod,
1.6 Acetoxy,
1.7 ($C_1$-$C_6$)-Alkylcarboxyamid,
1.8 Sulfonamid,
1.9 Nitro,
1.10 ($C_1$-$C_6$)-Alkyloxy oder
1.11 Amino,

d) Verbindung der Formel IV,

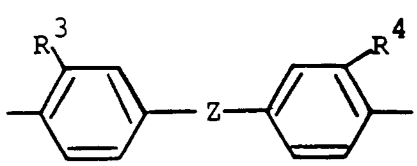

(IV)

worin $R^3$ oder $R^4$ unabhängig voneinander gleich oder verschieden sein können und jeder für
1) Wasserstoff oder
2) inerter Rest bedeuten und

Z für eine Verbindung aus der Gruppe
1) -O-,
2) -S-,
3)

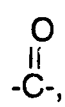

4) -$SO_2$-,
5)

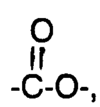

6) $-C_nH_{2n}-$, worin n eine ganze Zahl von 1 bis 10 bedeutet und die Kohlenstoffkette gerade oder verzweigt sein kann, oder

7) -O-Aryl-O, worin Aryl wie in c) definiert ist, steht,

e) Verbindung der Formel II,

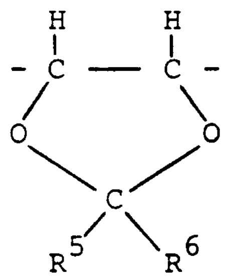

(II)

worin $R^5$ oder $R^6$ unabhängig voneinander

1) Wasserstoff,

2) Alkyl oder Alkenyl mit bis zu 18 C-Atomen,

3) Alkyl oder Alkenyl mit bis zu 18 C-Atomen, ein- oder mehrfach substituiert wie in b) 1 bis 16 definiert,

4) Aryl, mit 5 bis 14 Kohlenstoffatomen,

5) Aryl, mit 5 bis 14 Kohlenstoffatomen, ein- oder mehrfach substituiert wie in c) 1.1 bis 1.11 definiert, bedeuten oder

f) Verbindung der Formel V,

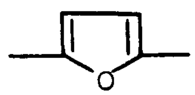

(V)

steht

wobei X für

a) -O-,

b) -NH- oder

c) -S-

steht,

wobei $R^2$ für

a) Verbindung der Formel III,

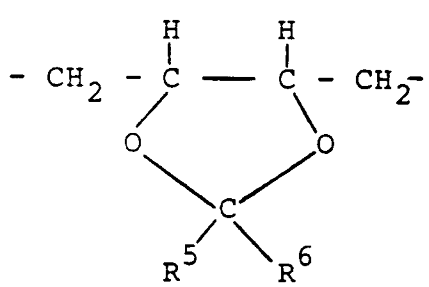

(III)

worin $R^5$ oder $R^6$ die obengenannten Bedeutungen haben,

b) Verbindung der Formel VI,

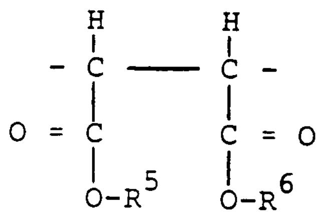

(VI)

worin $R^5$ oder $R^6$ die obengenannten Bedeutungen haben,

c) geradkettig oder verzweigtes Alkyl oder Alkenyl, mit 1 bis 18 C-Atomen,

d) geradkettig oder verzweigtes Alkyl oder Alkenyl, mit 1 bis 18 C-Atomen, ein- oder mehrfach substituiert durch einen Rest aus der Gruppe,

1) Carboxy,

2) Carboxy, worin die Hydroxygruppe ersetzt ist durch einen Rest aus der Gruppe,

2.1 -O- ($C_1$-$C_8$)-Alkyl,

2.2 Halogen, wie Fluor oder Chlor,

2.3 -O- ($C_1$-$C_4$)-Alkylcarbonyl,

2.4 ($C_1$-$C_6$)-Alkylamino,

2.5 ($C_1$-$C_6$)-Alkylamino, ein- oder mehrfach substituiert im Alkylteil durch einen Rest aus der Gruppe,

2.5.1 Hydroxy,

2.5.2 Mercapto,

2.5.3 -O- ($C_2$-$C_4$)-Alkyl,

2.6 genetisch kodierbare L-Aminosäure,

2.7 genetisch kodierbare L-Aminosäure, ein- oder mehrfach substituiert durch,

2.7.1 ($C_1$-$C_4$)-Alkyl,

3) Hydroxy,

4) Halogen, wie Fluor, Chlor, Brom oder Jod,

5) Amino,

6) Mercapto oder

7) Alkyl mit bis zu 8 C-Atomen,

e) Cyclische Verbindung der Formel VII,

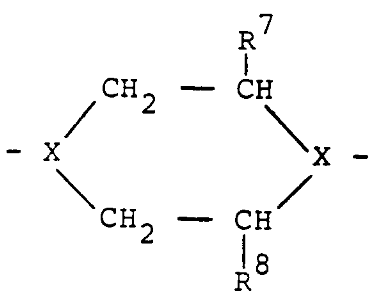

(VII)

worin $R^7$ oder $R^8$, unabhängig voneinander

1) Wasserstoff oder

2) ($C_1$-$C_3$)-Alkyl bedeuten

und X für N steht, oder

f) Aryl, mit 5 bis 14 Kohlenstoffatomen gegebenenfalls ein- oder zweifach substituiert durch

1.1 ($C_1$-$C_6$)-Alkyl,

1.2 ($C_2$-$C_6$)-Alkenyl,

1.3 ($C_1$-$C_6$)-Alkylcarboxy,

1.4 Hydroxy,

1.5 Halogen, wie Fluor, Chlor, Brom oder Jod,

1.6 Acetoxy,

1.7 ($C_1$-$C_6$)-Alkylcarboxyamid,

1.8 Sulfonamid,

1.9 Nitro,
1.10 ($C_1$-$C_6$)-Alkyloxy oder
1.11 Amino,

mit der Maßgabe, daß mehr als 5 Mol-% der Reste $R^1$ und/oder $R^2$ in dem Polykondensat der Formel I enthalten sind, wobei $R^1$ für eine Verbindung der Formel II und/oder $R^2$ für eine Verbindung der Formel III steht,
oder daß mehr als 5 Mol-% der Reste $R^1$ und $R^2$ in dem Polykondensat der Formel I enthalten sind, wobei
a) $R^1$ für eine Verbindung der Formel V und $R^2$ für eine Verbindung der Formel VI steht, oder
b) $R^1$ für Phenyl[1,2], Phenyl[1,3] oder Phenyl[1,4] und $R^2$ für eine Verbindung der Formel VI steht.

Besonders bevorzugt sind die Polykondensate der Formel I, die im wesentlichen mindestens 95 Mol-% an wiederkehrenden Struktureinheiten der Formel I enthalten, wobei $R^1$ für
a) eine Verbindung der Formel II,
worin $R^5$ oder $R^6$ unabhängig voneinander
1) Wasserstoff, mit der Maßgabe das $R^5$ und $R^6$ nicht gleichzeitig Wasserstoff bedeuten oder
2) geradkettiges oder verzweigtes Alkyl oder Alkylen mit bis zu 18 C-Atomen bedeuten,
b) Furo[2,5] oder
c) Phenyl[1,4], Phenyl[1,2], Phenyl[1,3], steht,
X für einen Rest aus der Gruppe -O- oder -NH- steht,
wobei $R^2$ für
a) eine Verbindung der Formel III,
worin $R^5$ oder $R^6$ die obengenannte Bedeutung haben,
b) eine Verbindung der Formel VI,
worin $R^5$ oder $R^6$ die obengenannte Bedeutung haben,
c) eine Verbindung der Formel IX,

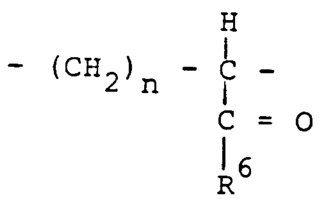

(IX)

wobei n für 3 oder 4 steht und $R^6$ die obengenannte Bedeutung hat,
mit der Maßgabe, daß mehr als 5 Mol-% der Reste $R^1$ und/oder $R^2$ in dem Polykondensat der Formel I enthalten sind, wobei $R^1$ für eine Verbindung der Formel II und/oder $R^2$ für eine Verbindung der Formel III steht,
oder daß mehr als 5 Mol-% der Reste $R^1$ und $R^2$ in dem Polykondensat der Formel I enthalten sind, wobei
a) $R^1$ für eine Verbindung der Formel V und $R^2$ für eine Verbindung der Formel VI steht, oder
b) $R^1$ für Phenyl[1,2], Phenyl[1,3] oder Phenyl[1,4) und $R^2$ für eine Verbindung der Formel VI steht.

Zur Herstellung der obengenannten Polykondensate setzt man ein Dicarbonsäuredichlorid der Formel X,

ClOC - $R^1$ - COCl (X)

mit einem oder mehreren der Diole, Diamine, Dithioverbindungen oder Diazine der Formeln XI, XII, XIII oder XIV um,

HO - $R^2$ - OH (XI)

$H_2N$ - $R^2$ - $NH_2$ (XII)

HS - $R^2$ - SH (XIII)

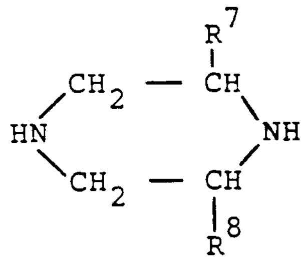

(XIV)

wobei $R^1$, $R^2$, $R^7$ und $R^8$ die obengenannte Bedeutung haben.

Das Dicarbonsäuredichlorid der Formel X und die einzelnen Diol-, Diamin-, Dithio- oder Diazintypen der Formeln XI, XII, XIII oder XIV können auch in Form von Mischungen miteinander eingesetzt werden.

Es ist für den Fachmann selbstverständlich, daß die Summe aller von den Dicarbonsäuren (A) abgeleiteten Struktureinheiten und die Summe aller von den Diolen, Dithioverbindungen, Diaminen und Diazinen (B) abgeleiteten Struktureinheiten im wesentlichen gleich sind, d. h., daß sie sich maximal um ca. 1 %, vorzugsweise maximal um 0,2 %, unterscheiden, insbesondere im Rahmen der praktischen Meß-und Dosierungsmöglichkeiten gleich sind.

Das Molekulargewicht der entstehenden Polyamide läßt sich unter anderem über die Auswahl der Mengenverhältnisse von A zu B steuern. Diese Auswahlkriterien sind dem Fachmann auf dem Gebiet der Polykondensation bekannt.

Beispiele für geeignete Dicarbonsäuren, von denen sich die Dicarbonsäuredichloride der Formel X ableiten, sind 2,3-O-Isopropyliden-L-weinsäure,
2-Chlorterephthalsäure, Furandicarbonsäure, 2-Bromterephthalsäure,
2-Methylterephthalsäure und insbesondere 2,3-O-Isopropyliden-L-weinsäure.

Beispiele für geeignete Diamine der Formel XII sind Lysinmethylester,
Naphthalin-1,4-diamin, Naphthalin-1,5-diamin, Naphthalin-2,6-diamin und insbesondere Lysinmethylester oder Diazine.

Beispiele für geeignete Diole der Formel XI sind Weinsäurediethylester,
2,3-O-Isopropyliden-L-threitol oder Weinsäurediisopropylester.

Die Kondensation der oben beschriebenen Komponenten wird im allgemeinen in Lösung ausgeführt.

Dazu werden die miteinander umzusetzenden monomeren Verbindungen in der Regel in einem organischen Lösungsmittel gelöst. Das organische Lösungsmittel enthält dabei vorzugsweise zumindest ein Lösungsmittel, wie z. B. N-Methyl-2-pyrrolidin,
N,N-Dimethylacetamid, Pyridin, Tetramethylharnstoff, N-Methyl-2-piperidon, Dichlormethan, N,N'-Dimethylethylenharnstoff, N-Methylcaprolactam,
N-Acetylpyrrolidin, N,N-Dimethylpropylenharnstoff. Für das erfindungsgemäße Verfahren sind die bevorzugten organischen Lösungsmittel Pyridin, Dichlormethan, Furan oder eine Mischung dieser Verbindungen von Bedeutung.

Bei einer bevorzugten Form der Durchführung der Lösungspolymerisation werden die Monomeren 2,3-O-Isopropyliden-L-weinsäuredichlorid und Weinsäurediethylester in einer Mischung von Pyridin und Dichlormethan unter heftigem Umrühren gemischt.

Die Polykondensationstemperaturen liegen bei der Lösungspolymerisation üblicherweise zwischen -20 °C und +120 °C, bevorzugt zwischen +10 °C und +100 °C. Besonders gute Ergebnisse werden bei Reaktionstemperaturen zwischen +10 °C und +80 °C erzielt.

Die Summe der Konzentrationen der monomeren Verbindungen in der Polymerisationsgemischlösung kann unter Beachtung des gewünschten Polymerisationsgrades, der gewünschten Viskosität des Polymerisationsgemisches, der Art der verwendeten monomeren Verbindungen, der Art des verwendeten Lösungsmittels und der gewünschten Polymerisationstemperatur eingestellt werden. Die günstigste Summe der Konzentrationen kann dabei aufgrund einer Reihe von Vorversuchen für den Ablauf der Polymerisation ermittelt werden.

Polykondensationsreaktionen werden vorzugsweise so ausgeführt, daß nach Abschluß der Reaktion 2 bis 15, vorzugsweise 5 bis 15 Gew.-% an Polykondensat in der Lösung vorliegen. Besonders gute Ergebnisse werden bei Konzentrationen von 5,0 bis 10 Gew.-% erzielt.

Im Verlauf der Polykondensation wächst das Molekulargewicht des Polymers und damit auch die Viskosität des Reaktionsansatzes an. Es werden Molekulargewichte von 500 bis 1000000 erreicht, bevorzugt 3.000 bis 100.000. Es werden in der Regel Viskositäten von mehr als 0,1 dl•g (Staudinger-Index, Dimethylformamid, 25 °C) erreicht.

Wenn die Polymerlösung die zur Weiterverarbeitung erforderliche Viskosität erreicht hat, kann die Polykondensation in üblicher Weise durch Zugabe von monofunktionellen Verbindungen, wie z. B. Acetylchlorid gestoppt werden. Anschließend kann der entstandene und locker an das Amidlösungsmittel gebundene Chlorwasserstoff durch Zugabe basischer Substanzen neutralisiert werden. Geeignet sind dafür beispielsweise Pyridin, Triethylamin, Morpholin, insbesondere aber Pyridin.

Die als Ausgangssubstanz einsetzbaren 2,3-O-Alkyliden-L-Weinsäuredichloride erhält man beispielsweise durch Umsetzung von Weinsäurealkylester mit
2,2-Dimethoxypropan zu den entsprechenden Weinsäuredialkylesteracetonketalen analog den von M. Carmack et al. (J. Org. Chem. 33, 1968, Seiten 2171 - 2173) beschriebenen Umsetzungen. Diese Weinsäuredialkylesteracetonketale werden mit Alkalihydroxiden in die entsprechenden Salze überfuhrt und abschließend durch Umsetzung mit Phosphortrichlorid, Phosphorpentachlorid, Oxalylchlorid oder Thionylchlorid in die entsprechenden L-Weinsäuredichloridderivate überführt.

Die Herstellung von 2,3-O-Alkyliden-L-threitol erfolgt beispielsweise durch Umsetzung von Weinsäuredialkylesteracetonketal mit $LiAlH_4$ zu dem entsprechenden
2,3-O-Alkyliden-2-threitol (P. W. Feit, J. Med. Chem. 7, 1964, Seiten 14 - 17).

Furandicarbonsäuredichloride sind beispielsweise nach dem von Moore and Kelley (American Chemical Society, 11, No. 3, 1978, Seite 568 - 573) beschriebenen Verfahren erhältlich.

Ein weiterer Gegenstand der Erfindung sind Ultraschall-Kontrastmittel, bestehend aus Mikropartikeln, welche ein Gas und ein Polykondensat der Formel I enthalten.

Ein vorteilhaftes Verfahren zur Herstellung von Ultraschall-Kontrastmittel aus den erfindungsgemäßen Polykondensaten besteht darin, ein oder mehrere der Polykondensate der Formel I in einem Lösungsmittel oder Lösungsmittelgemisch mit hohem Schmelzpunkt zu lösen oder diese Derivate mit einem oder mehreren weiteren Polymeren und/oder physiologisch unbedenklichen Hilfsstoffen zu mischen und in einem Lösungsmittel oder Lösungsmittelgemisch mit hohem Schmelzpunkt zu lösen und in ein kondensiertes kaltes Gas, z. B. flüssigen Stickstoff, zu vertropfen. Durch das Leidenfrostsche Phänomen entstehen dabei runde Partikel. Als Lösungsmittel sind beispielsweise Dimethylformamid, Dimethylsulfoxid, Dioxan oder Mischungen mit Alkoholen einsetzbar. Das hochschmelzende und mit Wasser mischbare Lösungsmittel wird z. B. durch Überführung der Mikropartikel in Wasser herausgelöst und das Polymere dabei ausgefällt, wobei die Kugelgestalt der Mikropartikel erhalten bleibt.

Besitzt das verwendete organische Lösungsmittel neben einem hohen Schmelzpunkt zugleich einen niedrigen Siedepunkt, so läßt sich dieses Vertropfungsverfahren weiter vereinfachen, indem das Lösungsmittel, z. B. Dioxan, direkt mittels Gefriertrocknung schonend entfernt werden kann.

Ein weiteres Verfahren zur Herstellung von Ultraschall-Kontrastmittel aus den erfindungsgemäßen Polymer besteht darin, ein oder mehrere der Polykondensate der Formel I in einem Lösungsmittel oder Lösungsmittelgemisch aufzulösen und gegebenenfalls nach Zusatz eines weiteren Lösungsmittels und/oder eines oder mehreren weiteren Polymeren auszufällen oder in Wasser zu dispergieren. Als weitere Polymere eignen sich beispielsweise Polyvinylalkohol (®Mowiol 28-99) oder Polyoxyethylenpolyoxypropylen (®Pluronic F 127). Als weiteres Lösungsmittel können z. B. Ether verwendet werden. Mikropartikel mit einem Durchmesser von 0,5 bis 15 $\mu$m erhält man durch starkes Rühren z. B. mit einem Mixer (25000 UPM). Anschließend werden die Lösungsmittel z. B. durch lyophilisieren entfernt.

Ein besonders vorteilhaftes Verfahren besteht darin, die Mikropartikel durch Sprühtrocknung zu gewinnen. Dazu werden ein oder mehrere Verbindungen der Formel I gelöst oder diese Verbindungen werden mit einem oder mehreren weiteren Polymeren und/oder physiologisch unbedenklichen Hilfsstoffen gemischt und in Lösung gebracht. Als Lösungsmittel oder Lösungsmittelgemische eignen sich beispielsweise Tetrahydrofuran, Methylenchlorid, Furan, DMSO (Dimethylsulfoxid), Dioxan oder Aceton. Anschließend wird die Lösung in einem Sprühtrockner zu Mikropartikeln versprüht.

Bei dem beschriebenen Verfahren können die Polymere der Formel I allein oder auch als Gemisch verschiedener Polymere der Formel I verwendet werden. Diese Polymere können auch in Mischungen mit anderen bioabbaubaren und/oder bioverträglichen Polymeren (z. B. ®Pluronic F68, PHEA, Dextrane, Polyethylenglykole, Hydroxyethylstärke und andere abbaubare oder ausscheidbare Polysaccharide) oder physiologisch unbedenklichen Hilfsstoffen (z. B. Polymerweichmacher oder DMSO) eingesetzt werden.

Die Mikropartikel enthalten Gase beispielsweise Luft, Stickstoff, Edelgase wie Helium, Neon, Argon oder Krypton, Wasserstoff, Kohlendioxid, Sauerstoff oder deren Gemische. Die Mikropartikel werden mit einem Gas beladen, indem beispielsweise die Mikropartikel nach der Lyophilisierung in einer entsprechenden Gasatmosphäre gelagert oder bei der Sprühtrocknung direkt bei der Herstellung in einer entsprechenden Gasatmosphäre gewonnen werden.

Die Erfindung betrifft ferner ein Diagnostikum oder Therapeutikum enthaltend mindestens ein erfindungsgemäßes Ultraschall-Kontrastmittel neben physiologisch annehmbaren und pharmakologisch geeigneten Trägern und gegebenenfalls weiteren Zusatz- und/oder Hilfsstoffen.

Die Ultraschall-Kontrastmittel werden vor Applikation durch Zusatz von einem oder mehreren physiologisch annehmbaren Trägern und gegebenenfalls weiteren Zusatz-und/oder Hilfsstoffen in eine geeignete diagnostische oder therapeutische Darreichungsform überführt. Die Ultraschall-Kontrastmittel werden beispielsweise vor Applikation durch Zusatz von Wasser und Mischen suspendiert.

Durch Zugabe osmotisch aktiver Substanzen, beispielsweise Kochsalz, Galaktose, Glukose, Fruktose, kann die physiologische Isotonie der Partikelsuspension hergestellt werden.

Bei den beschriebenen Verfahren zur Herstellung der Ultraschall-Kontrastmittel kann man Partikelgrößen erreichen bei denen 90 % der Partikel zwischen 0,1 µm und 10 µm liegen. Mit dem Sprühtrocknungsverfahren können Partikelgrößenverteilungenerreicht werden, bei denen 90 % der Partikel kleiner als 3 µm sind. Größere Teilchen werden durch Aussieben, beispielsweise mit einem 15 µm Siebgewebe und/oder 3 µm Siebgewebe entfernt. Bei der Verwendung dieser Mikropartikel als Ultraschall-Kontrastmittel zur Diagnose von Herz-Kreislauf-Erkrankungen haben sich Partikelgrößen von 0,1 µm bis 7 µm bewährt, vorteilhaft werden Partikelgrößen von 0,1 µm bis 3 µm eingesetzt. Die Ultraschall-Kontrastmittel werden beispielsweise in die Blutbahn injiziert. Je Injektion werden 0,1 mg bis 1000 mg der Mikropartikel, bevorzugt 1 mg bis 100 mg, eingesetzt.

Die im vorstehenden beschriebenen Ultraschall-Kontrastmittel können sowohl für diagnostische als auch therapeutische Verfahren verwendet werden. Die Verwendung der erfindungsgemäßen Ultraschall-Kontrastmittel ist nicht nur auf die Sichtbarmachung des Blutstroms im rechtventtrikuläuren Teil des Blutkreislaufes nach venöser Applikation beschränkt. Die Ultraschall-Kontrastmittel können mit ausgezeichnetem Erfolg für die Untersuchung der linken Herzseite verwendet werden. Ferner ist es auch möglich andere vom Blut versorgte Organe wie Leber, Milz, Niere, Gehirn oder Myocard mit diesen Kontrastmitteln sichtbar zu machen.

Die Ultraschall-Kontrastmittel eignen sich aber auch zum Sichtbarmachen von Hohlräumen in Menschen, Tieren oder Pflanzen, beispielsweise Harnblase, Harnleiter, Gebärmutter oder Vagina.

Einige der erfindungsgemäßen Polyamide sind thermoplastisch und eignen sich daher zur Herstellung von Wirkstoffdepotformen nach verschiedenen Methoden, wie z. B. durch Kompression, Extrusion, Fällung, Versprühung, etc.

Aus den erfindungsgemäßen Polykondensaten können nach bekannten Methoden implantierbare Partikel, insbesondere Mikrokapseln und Mikrosphären sowie durch Kompaktierung makroskopische Formkörper beliebiger Geometrie, insbesondere Tabletten und Stäbchen hergestellt werden. Abbauversuche in vitro mit den erfindungsgemäßen Polykondensaten der Formel I haben gezeigt, daß der Abbau über funktionelle Seitengruppen, Ringöffnung und Hauptkettenabbau verläuft.

In den folgenden Beispielen wird die Erfindung im einzelnen beschrieben. Prozentangaben beziehen sich auf das Gewicht, sofern nicht anders angegeben.

Beispiel 1

Dimethyl-2,3-O-isopropyliden-L-tartrat

Eine Mischung aus 101 g (0,673 mol) L-Weinsäure, 160 g (1,54 mol)
2,2-Dimethoxypropan (Aldrich), 40 ml wasserfreiem Methanol und
0,4 g p-Toluolsulfonsäuremonohydrat wird für 1,5 Stunden unter Rückfluß vorsichtig erwärmt und gerührt. Die dunkelrote homogene Lösung wird zusätzlich mit 79,5 g (0,764 mol) 2,2-Dimethoxypropan und 450 ml Cyclohexan versetzt. Das entstehende zweiphasige Gemisch wird unter Rückfluß erwärmt und die Aceton-Cyclohexan-sowie die Methanol-Cyclohexan-Azeotrope werden vorsichtig entfernt. Nach 47 Stunden wurden 590 ml Destillat erhalten. Mit wasserfreiem Kaliumcarbonat (1 g) wird neutralisiert. Das Lösungsmittel wird abdestilliert und es werden 148,3 g
Dimethyl-2,3-O-isopropyliden-L-tartrat als hellgelbe Flüssigkeit erhalten. Siedetemperatur 82 - 90 °C; $[\alpha]_D^{20}$ - 49 °.

Beispiel 2

2,3-O-Isopropyliden-L-threitol

Eine Suspension von $LiAlH_4$ (42 g) in Diethylether (400 ml) wird während kräftigem Rühren unter Rückfluß gekocht. Eine Lösung von
Dimethyl-2,3-O-isopropyliden-L-tartrat (123 g) aus Beispiel 1 in Diethylether (500 ml) wird tropfenweise ohne

erhitzen über 2 Stunden zu der $LiAlH_4$ Suspension zugegeben. Nach weiteren 3 Stunden Erhitzen wird Ethylacetat (30 ml) zugefügt und die Reaktionslösung wird auf 0 -5 °C gekühlt. Nach der Zugabe von Wasser (42 ml), 4N, NaOH (42 ml) und Wasser (130 ml) wird der ausgefallene Rückstand durch Filtration entfernt und mit Ether extrahiert. Die Etherextrakte werden vereinigt, über wasserfreiem $MgSO_4$ getrocknet und der Ether wird anschließend verdampft. Destillation des Rückstandes ergibt 64 g 2,3-O-Isopropyliden-L-threitol;
Siedepunkt 96 - 96,5 °C; $[\alpha]_D^{20}$ + 4,1 °.

Beispiel 3

2,3-O-Isopropyliden-L-dikaliumtartrat

20 g Weinsäuredimethylesteracetonketal (91 mmol), 15 g KOH p. a. (270 mmol), 60 ml destilliertes Wasser und 120 ml Ethanol (p. a.) werden unter Rückfluß erhitzt. Nach ca. 4 Stunden wird die Reaktionslösung eingeengt, der Rückstand in wenig Wasser gelöst und in eisgekühltem Ethanol ausgefällt. Nach einer zweiten Fällung wird der Ansatz bis zur Gewichtskonstanz getrocknet.

Weiße, kristalline Substanz; Ausbeute: 23 g entspr. 95 % d. Th.

$^1$H-NMR (100 MHz) in $D_2O$: Singulett 1,43 ppm, 6H, $-CH_3$
Singulett 4,46 ppm, 2H, $-\overline{CH}$

Beispiel 4

2,3-O-Isopropyliden-L-weinsäuredichlorid

15 g 2,3-O-Isopropyliden-L-dikaliumtartrat (56 mmol) werden vorsichtig (starke Wärmeentwicklung) mit 30 ml Thionylchlorid, das über Baumwollsamenöl zuvor destilliert wurde, umgesetzt. Beim Rühren über Nacht verfestigt sich der Ansatz, aus dem anschließend das überschüssige Thionylchlorid abdestilliert wird. Im Ölpumpenvakuum erfolgt dann die Destillation des Säurechlorides ($Sdp._{0,01\ bar}$: 75 - 80 °C).

Farblose Flüssigkeit; Ausbeute: 8,3 g entspr. 65 % d. Th.

| Elementaranalyse: | % C | % H | % Cl | % S |
|---|---|---|---|---|
| ber. | 37.0 | 3.5 | 31.3 | 0 |
| gef. | 37.2 | 3.6 | 31.4 | <0,3 |

$^1$H-NMR (100 MHz) in $CDCl_3$: Singulett 1,50 ppm, 6H, $-CH_3$
Singulett 5,18 ppm, 2H, $-\overline{CH}$

Beispiel 5

Poly-(2,3-O-isopropyliden)-L-weinsäure-(2', 3'-(1', 4'-diethyl)-L-tartryl)ester

a) 2,88 g 2,3-O-Isopropyliden-L-weinsäuredichlorid (12,7 mmol), 2,61 g Weinsäurediethylester (12,7 mmol), 2,1 ml Pyridin und 50 ml getrocknetes Dichlormethan werden gemischt und über 3 Tage gerührt. Es bildet sich währenddessen ein weißer Niederschlag. Zur Vervollständigung der Reaktion wird noch 1 Tag unter Rückfluß erhitzt und anschließend in trockenem Diisopropylether bei -20 °C gefällt. Nach Absaugen und Trocknen wird in Tetrahydrofuran (THF) gelöst und in Wasser gefällt, erneut abgesaugt und im Vakuum getrocknet.

Weißes Pulver; Ausbeute: 3,4 g entspr. 78 % d. Th.

| Elementaranalyse: | % C | % H |
|---|---|---|
| ber. | 50.0 | 5.5 |
| gef. | 49.4 | 5.5 |

$^1$H-NMR (300 MHz) in $CDCl_3$: Triplett 1,26 ppm, 6H, $-CH_2\underline{CH_3}$
Singulett 1,45 ppm, 6H, $-\overline{CH_3}$

Quartruplett 4,20 ppm, 4H, $-\underline{CH_2}-CH_3$
Singulett 5,05 ppm, 2H
Singulett 5,82 ppm, 2H

Molekulargewicht 15 000 ($M_W$) (nach vorangegangener Eichung einer Gelpermeationschromatography (GPC) mittels Oligomeren):

b) 2,59 g 2,3-O-Isopropyliden-L-weinsäuredichlorid (11,4 mmol), und 2,87 g Weinsäurediethylester (13,9 mmol) werden wie in a) miteinander umgesetzt. Das Endprodukt hat ein Molekulargewicht von 3500 (bestimmt mittels GPC).

c) Viskositätsmessungen

Die Viskositätsmessung wird mit einem Mikro-Ubbelohde Kapillarviskosimeter der Firma Schott durchgeführt. Zur Bestimmung des Staudinger Indexes wird konzentrationsabhängig zwischen 0,01 und 0,1 g/dl die relativen Viskositäten, wie bei Schacht et al. in Dimethylformamid bei 25°C gemessen. Unter Berücksichtigung eines als "Wandadsorbtion" diskutierten Effektes (B. Vollmert, Grundriß der Makromolekularen Chemie, E. Vollmert-Verlag Karlsruhe Bd. III, S. 70, 1982) ergibt sich als Viskosität von Poly-(2,3-O-isopropyliden)-L-weinsäure-(2',3'-(1',4'-diethyl)-L-tartryl)-ester hergestellt wie nach Beispiel 5 a) mit einem gelpermeationschromatographisch ermittelten Molekulargewicht von 15 000 ein Staudinger-Index von (n) = 0,9 dl/g und bei einem Oligomer von $M_{gpc}$ = 3500 (Beispiel 5b)) (n) = 0,5 dl/g. Das letztgenannte Oligomer hatte einen Polymerisationsgrad von ca. 10. Das schließt aus, daß unter den beschriebenen Bedingungen ein Oligomer mit einer Viskosität von 0,04 dl/g existiert.

Beispiel 6

Poly-(2,3-O-isopropyliden)-L-weinsäure-(2',3'-O-isopropyliden-L-threityl)ester

5,20 g 2,3-O-Isopropyliden-L-weinsäuredichlorid (22,9 mmol),
3,71 g 2,3-O-Isopropyliden-L-threitol (22,9 mmol), 3,7 ml Pyridin und
100 ml getrocknetes Dichlormethan werden gemischt und analog Beispiel 5 aufgearbeitet.

Leicht gefärbtes Produkt; Ausbeute: 5,7 g entspr. 80 % d. Th.

Molekulargewicht 65000 (nach vorangegangener Eichung unter GPC mittels Oligomeren): ($M_W$)

$^1$H-NMR (300 MHz) in $CDCl_3$: Singulett 1,41 ppm, 6H, $-CH_3$
Singulett 1,48 ppm, 6H, $-CH_3$
Singulett 4,10 ppm, 2H, -CH
Multiplett 4,35 ppm, 4H, $-CH_2$
Singulett 4,87 ppm, 2H, -CH

Beispiel 7

Poly-(2,3-O-isopropyliden)-L-weinsäure-(2',3'-(1',4'-diisopropyl)-L-tartryl)ester

10,48 g 2,3-O-Isopropyliden-L-weinsäuredichlorid (46,3 mmol),
10,8 g L-Weinsäurediisopropylester (46,2 mmol), 7,5 ml Pyridin und 200 ml getrocknetes Dichlormethan werden gemischt und analog Beispiel 5 weiter aufgearbeitet.

Ausbeute: 11,3 g entspr. 63 % d. Th.

Beispiel 8

Polyfurandicarbonsäure-(2,3-(1,4-diethyl)-L-tartryl)ester

12,01 g Furandicarbonsäuredichlorid (62,2 mmol), 12,82 g Weinsäurediethylester (62,2 mmol), 10 ml getrocknetes Pyridin und 200 ml getrocknetes Dichlormethan werden gemischt und analog Beispiel 5 weiter bearbeitet.

Weißes Pulver; Ausbeute: 15 g entspr. 74 % d. Th.

$^1$H-NMR (300 MHz) in $CDCl_3$: Triplett 1,22 ppm, 6H, $-CH_3$
Ouartruplett 4,27 ppm, 4H, $-\underline{CH_2}-CH_3$
Singulett 6,00 ppm, 2H, -CH
Singulett 7,35 ppm, 2H, $=CH_{arom.}$

Beispiel 9

Poly-(2,3-(1,4-diethyl)-L-tartryl)-terephthalat

8,81 g Terephthalsäuredichlorid (43,4 mmol), 8,95 g Weinsäurediethylester (43,4 mmol), 7 ml Pyridin und 180 ml getrocknetes Dichlormethan werden gemischt und analog Beispiel 6 weiter bearbeitet.

Ausbeute: 13,6 g entspr. 94 % d. Th.

$^1$H-NMR (300 MHz) in $CDCl_3$:
Triplett 1,19 ppm, 6H, $-CH_3$
Quartruplett 4,22 ppm, 4H, $-\underline{CH_2}-CH_3$
Singulett 6,03 ppm, 2H, $-\underline{CH}$
Singulett 8,20 ppm, 4H, $=CH_{arom.}$

Beispiel 10

Polylysinmethylester-2,3-O-isopropyliden-L-weinsäureamid

Es werden 5,3 g $Na_2CO_3$ in 70 ml Wasser, 3,0 g Lysinmethylester in 20 ml Wasser und 1 ml 2,3-O-Isopropyliden-L-weinsäuredichlorid in 90 ml Dichlormethan gelöst. Die beiden wässrigen Lösungen werden zusammengegeben und heftig gerührt sowie unmittelbar darauf die organische Phase hinzugefügt. Es bildet sich langsam ein Niederschlag. Nach 10 Minuten wird das Dichlormethan abrotiert und der Niederschlag mit Wasser mehrfach gewaschen.

Ausbeute: 1,1 g.

$^1$H-NMR (100 MHz) in DMSO-$d_6$:
breites Signal um 1,35 ppm, 10H, $-CH_3$, $-NHCH_2-\underline{CH_2}-\underline{CH_2}$
breites Signal um 1,70 ppm, 2H, $-\underline{CH_2}-CH$
breites Signal um 3,08 ppm, 2H, $-NH-\underline{CH_2}$
Singulett 3,61 ppm, 3H, $-OCH_3$
Multiplett 4 - 4,7 ppm, 3H, -CH
breite Signale 7,6 - 8,5 ppm, 2H, -CONH

Beispiel 11

Herstellung von wirkstoffhaltigen Mikropartikeln

4 g Poly-(2,3-O-isopropyliden)-L-weinsäure(2',3'-(1',4'-diethyl)-L-tartryl)ester (Beispiel 5) werden in den in Tabelle 1 angegebenen Lösungsmitteln zu 10 % gelöst und mit 0,2 g des Peptidwirkstoffes Buserelin, gelöst in 1 ml Wasser, versetzt. Anschließend werden die Suspensionen/Lösungen in einem Sprühtrockner (Mini Spray Dryer Büchi 190, W.-Germany) zu Mikropartikel versprüht. Typische Sprühparameter sind in Tabelle 2 aufgestellt.

Tabelle 1

| Lösungsmittel | Partikelgröße | Wirkstoffbeladung | Initiale Wirkstofffreisetzung |
|---|---|---|---|
| THF | 8 µm | 4 % | 55 % |
| $CH_2Cl_2$ | 4 µm | 3,6 % | 25 % |
| Furan | 5 µm | 3,4 % | 20 % |
| Aceton | 3 µm | 4,2 % | 10 % |

Tabelle 2

| | | | |
|---|---|---|---|
| Eingangstemperatur | 61 °C | Druck Düsenluft | 2,8 bar |
| Ausgangstemperatur | 45 °C | Fluß Düsenluft | 300 Nl/h |
| Heizung | 2,2 Skalenteile | Druck im Filter | -25 mbar |
| Pumpenstellung | 10 | Aspiratorstellung | 10 |

Eine jeweils 3,6 µg Buserelin äquivalente Polymermenge (85 - 100 mg) werden in einem Suspensionshilfsmittel, bestehend aus 150 mg Dextran 40 (Firma Roth, W.-Germany), 7,5 mg Polysorbat und 13,5 mg

NaCl in 1,5 ml destilliertem Wasser dispergiert. Die Suspension wird vollständig durch ein 15 µm Siebgewebe gedrückt und anschließend lyophilisiert. Vor der Applikation werden die Mikropartikel mit Wasser resuspendiert.

Die Größe der Mikropartikel ist in einem Cilas-Granulometer 715, die Wirkstoffbeladung mittels HPLC und die Initiale Wirkstofffreisetzung mit Hilfe eines in vitro-Tests bestimmt worden. Für den in vitro-Test wurden 20 mg Mikropartikel in 5 ml Suspensionshilfsmittel (s. o.) dispergiert und in Rollrandfläschen mit Magnetkern 24 Stunden heftig rührend in Suspension gehalten. Anschließend wird abfiltriert und im Filtrat die aus den Mikropartikeln ausgetretene Menge an Wirkstoff bestimmt.

Beispiel 12

Herstellung von stäbchenförmigen Implantaten ("rods")

Ein inniges Gemisch aus pulverförmigen, erfindungsgemäßen Polykondensaten, Additiven und Wirkstoffe(n) wird in einer geeigneten Vorrichtung, z. B. einem Extruder für Thermoplasten, über den Erweichungspunkt erhitzt, wobei eine verformbare Masse entsteht. Durch Kneten werden Additive und Wirkstoff(e) im erweichten Polymer homogen dispergiert und die erhaltene Polymer/Wirkstoff-Suspension durch eine Düse geeigneten Durchmessers (> 0,5 mm) gepreßt. Beim Abkühlen erstarrt der Strang der extrudierten Polymer/Wirkstoff-Suspension zu einem festen, stäbchenförmigen Aggregat, dessen Wirkstoffgehalt von seiner Länge und seinem Durchmesser bestimmt ist.

**Patentansprüche**

1. Polykondensat, das mindestens 95 Mol-% an wiederkehrenden Struktureinheiten der Formel I enthält,

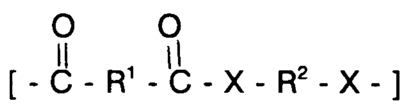

(I)

wobei $R^1$ für

a) Verbindung der Formel II

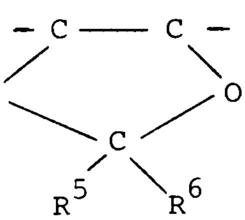

(II)

worin $R^5$ und $R^6$ inerte Reste bedeuten,

b) geradkettiges oder verzweigtes Alkyl, Alkenyl, Cycloalkyl oder Cycloalkenyl, die mit einem oder mehreren inerten Resten substituiert sein können,

c) ein- oder mehrkerniges Aryl, ein oder mehrfach substituiert durch inerte Reste,

d) Verbindung der Formel V,

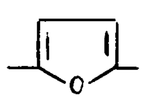

(V)

e) heterocyclischer Rest, der ein- oder mehrkernig und ein- oder mehrfach durch inerte Reste substituiert sein kann steht,

wobei X für

a) -O-,

b) -NH- oder
c) -S-
steht,

wobei $R^2$ für
a) Verbindung der Formel III,

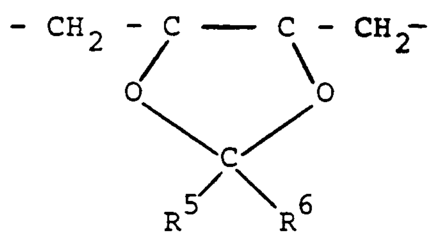

(III)

worin $R^5$ und $R^6$ die obengenannte Bedeutung haben,
b) geradkettiges oder verzweigtes Alkyl, Alkenyl, Cycloalkyl oder Cycloalkenyl, die mit einem oder mehreren inerten Resten substituiert sein können,
c) ein- oder mehrkerniges Aryl, ein- oder mehrfach substituiert durch inerte Reste,
d) heterocyclischer Rest, der ein- oder mehrkernig und ein- oder mehrfach durch inerte Reste substituiert sein kann, oder
e) Verbindung der Formel VI,

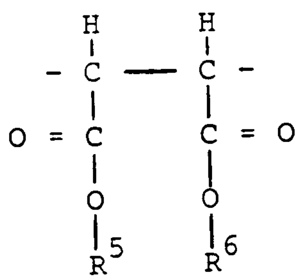

(VI)

worin $R^5$ und $R^6$ die obengenannte Bedeutung haben,
mit der Maßgabe, daß mehr als 5 Mol-% der Reste $R^1$ und/oder $R^2$ in dem Polykondensat der Formel I enthalten sind, wobei $R^1$ für eine Verbindung der Formel II oder $R^2$ für eine Verbindung der Formel III oder $R^1$ für eine Verbindung der Formel II und $R^2$ für eine Verbindung der Formel III steht,
oder daß mehr als 5 Mol-% der Reste $R^1$ und $R^2$ in dem Polykondensat der Formel I enthalten sind, wobei
a) $R^1$ für eine Verbindung der Formel V und $R^2$ für eine Verbindung der Formel VI steht, oder
b) $R^1$ für Phenyl[1,2], Phenyl[1,3] oder Phenyl[1,4] und
$R^2$ für eine Verbindung der Formel VI steht.

**2.** Polykondensat der Formel I nach Anspruch 1, wobei $R^1$ für
a) geradkettig oder verzweigtes $(C_1-C_{18})$-Alkyl oder geradkettig oder verzweigtes $(C_1-C_{18})$-Alkenyl, das eine oder mehrere Doppelbindungen enthält,
b) geradkettig oder verzweigtes $(C_1-C_{18})$-Alkyl oder geradkettig oder verzweigtes $(C_1-C_{18})$-Alkenyl, das eine oder mehrere Doppelbindungen enthält, ein- oder mehrfach substituiert durch
1) $(C_5-C_7)$-Cycloalkyl,
2) $(C_5-C_7)$-Cycloalkenyl, mit einer oder mehrerer Doppelbindungen,
3) Halogen, wie Fluor oder Chlor,
4) Mercapto,
5) Hydroxy,
6) $(C_1-C_6)$-Alkoxy,
7) Phenoxy,

8) Phenoxy, ein- oder mehrfach substituiert durch
8.1 Halogen, wie Fluor, Chlor, Brom oder Jod,
8.2 ($C_1$-$C_6$)-Alkyl,
8.3 ($C_1$-$C_6$)-Alkoxy,
8.4 Nitro oder
8.5 Carbethoxy,
9) Naphthyloxy,
10) Benzyloxy,
11) Benzyloxy, ein- oder mehrfach substituiert im Arylteil durch
11.1 Methoxy,
11.2 Carboxyamido,
11.3 Amino,
11.4 Halogen, wie Fluor, Chlor, Brom oder Jod,
11.5 Nitro oder
11.6 Methyl,
12) Amino,
13) Monoalkylamino, mit bis zu 7 C-Atomen,
14) Monoalkylamino, mit bis zu 7 C-Atomen, ein- oder mehrfach substituiert im Alkylteil durch
14.1 Hydroxy,
14.2 Carboxy,
14.3 Carboxyamid,
14.4 Carboethoxy,
14.5 Amino,
14.6 ($C_1$-$C_6$)-Alkylamino,
14.7 Di-($C_1$-$C_6$)-alkylamino,
14.8 Piperidin oder
14.9 Morpholin,
15) Dialkylamino, mit bis zu 7 C-Atomen, oder
16) Dialkylamino, mit bis zu 7 C-Atomen, substituiert wie in b) 14.1 bis 14.9 definiert,

c) Aryl, mit 5 bis 14 Kohlenstoffatomen, gegebenenfalls ein- oder zweifach substituiert durch
1.1 ($C_1$-$C_6$)-Alkyl,
1.2 ($C_2$-$C_6$)-Alkenyl,
1.3 ($C_1$-$C_6$)-Alkylcarboxy,
1.4 Hydroxy,
1.5 Halogen, wie Fluor, Chlor, Brom oder Jod,
1.6 Acetoxy,
1.7 ($C_1$-$C_6$)-Alkylcarboxyamid,
1.8 Sulfonamid,
1.9 Nitro,
1.10 ($C_1$-$C_6$)-Alkyloxy oder
1.11 Amino,

d) Verbindung der Formel IV,

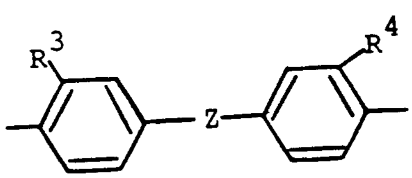

(IV)

worin $R^3$ oder $R^4$ unabhängig voneinander
1) Wasserstoff oder
2) inerter Rest bedeuten und

Z für eine Verbindung aus der Gruppe,
1) -O-,
2) -S-,
3)

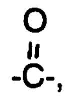

4) $-SO_2-$,

5)

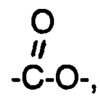

6) $-C_nH_{2n}-$, worin n eine ganze Zahl von 1 bis 10 bedeutet und die Kohlenstoffkette gerade oder verzweigt sein kann, oder

7) -O-Aryl-O, worin Aryl wie in c) definiert ist, steht,

e) Verbindung der Formel II,

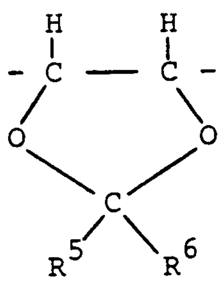

(II)

worin $R^5$ oder $R^6$ unabhängig voneinander

1) Wasserstoff,

2) Alkyl oder Alkenyl mit bis zu 18 C-Atomen,

3) Alkyl oder Alkenyl mit bis zu 18 C-Atomen, ein- oder mehrfach substituiert wie in b) 1 bis 16 definiert,

4) Aryl, mit 6 bis 14 Kohlenstoffatomen,

5) Aryl, mit 6 bis 14 Kohlenstoffatomen, ein- oder mehrfach substituiert wie in c) 1.1 bis 1.11 definiert, bedeuten oder

f) Verbindung der Formel V,

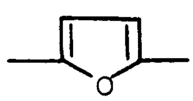

(V)

steht,

wobei X für

a) -O-,

b) -NH- oder

c) -S-

steht, wobei $R^2$ für

a) Verbindung der Formel III,

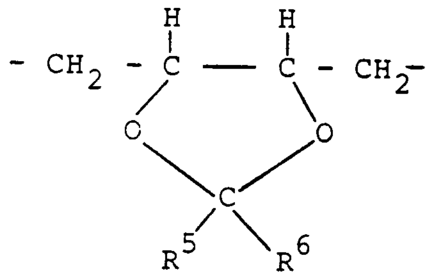

(III)

worin $R^5$ oder $R^6$ die obengenannten Bedeutungen haben,

b) Verbindung der Formel VI,

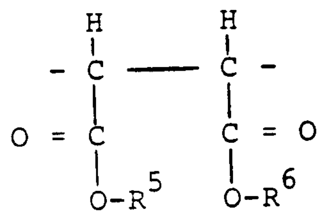

(VI)

worin $R^5$ oder $R^6$ die obengenannten Bedeutungen haben,

c) geradkettig oder verzweigtes Alkyl oder Alkenyl, mit 1 bis 18 C-Atomen,

d) geradkettig oder verzweigtes Alkyl oder Alkenyl, mit 1 bis 18 C-Atomen, ein- oder mehrfach substituiert durch einen Rest aus der Gruppe,

1) Carboxy,

2) Carboxy, worin die Hydroxygruppe ersetzt ist durch einen Rest aus der Gruppe,

2.1 -O- ($C_1$-$C_8$)-Alkyl,

2.2 Halogen, wie Fluor oder Chlor,

2.3 -O- ($C_1$-$C_4$)-Alkylcarbonyl,

2.4 ($C_1$-$C_6$)-Alkylamino,

2.5 ($C_1$-$C_6$)-Alkylamino, ein- oder mehrfach substituiert im Alkylteil durch einen Rest aus der Gruppe,

2.5.1 Hydroxy,

2.5.2 Mercapto oder

2.5.3 -O- ($C_2$-$C_4$)-Alkyl,

2.6 genetisch kodierbare L-Aminosäure,

2.7 genetisch kodierbare L-Aminosäure, ein- oder mehrfach substituiert durch,

2.7.1 ($C_1$-$C_4$)-Alkyl,

3) Hydroxy,

4) Halogen, wie Fluor, Chlor, Brom oder Jod,

5) Amino,

6) Mercapto oder

7) Alkyl mit bis zu 8 C-Atomen,

e) Cyclische Verbindung der Formel VII,

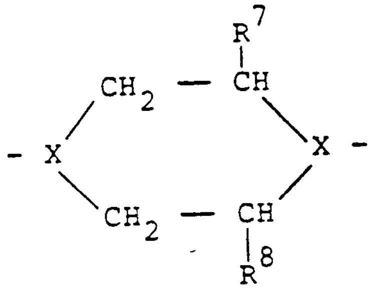

(VII)

worin $R^7$ oder $R^8$, unabhängig voneinander

1) Wasserstoff oder

2) ($C_1$-$C_3$)-Alkyl bedeuten

und X für N steht, oder

f) Aryl, mit 5 bis 14 Kohlenstoffatomen, gegebenenfalls ein- oder zweifach substituiert durch

1.1 ($C_1$-$C_6$)-Alkyl,

1.2 ($C_2$-$C_6$)-Alkenyl,

1.3 ($C_1$-$C_6$)-Alkylcarboxy,

1.4 Hydroxy,

1.5 Halogen, wie Fluor, Chlor, Brom oder Jod,

1.6 Acetoxy,

1.7 ($C_1$-$C_6$)-Alkylcarboxyamid,

1.8 Sulfonamid,

1.9 Nitro,

1.10 ($C_1$-$C_6$)-Alkyloxy oder

1.11 Amino,

mit der Maßgabe, daß mehr als 5 Mol-% der Reste $R^1$ und/oder $R^2$ in dem Polykondensat der Formel I enthalten sind, wobei $R^1$ für eine Verbindung der Formel II oder $R^2$ für eine Verbindung der Formel III oder $R^1$ für eine Verbindung der Formel II und $R^2$ für eine Verbindung der Formel III steht, oder daß mehr als 5 Mol-% der Reste $R^1$ und $R^2$ in dem Polykondensat der Formel I enthalten sind, wobei

a) $R^1$ für eine Verbindung der Formel V und $R^2$ für eine Verbindung der Formel VI steht, oder

b) $R^1$ für Phenyl[1,2], Phenyl[1,3] oder Phenyl[1,4] und $R^2$ für eine Verbindung der Formel VI steht.

**3.** Polykondensat, nach Anspruch 1 oder 2, wobei $R^1$ für

a) eine Verbindung der Formel II,

worin $R^5$ oder $R^6$ unabhängig voneinander

1) Wasserstoff, mit der Maßgabe das $R^5$ und $R^6$ nicht gleichzeitig Wasserstoff bedeuten oder

2) geradkettiges oder verzweigtes Alkyl oder Alkylen mit bis zu 18 C-Atomen bedeuten,

b) Furo[2,5] oder

c) Phenyl[1,4], Phenyl[1,2], Phenyl[1,3], steht,

X für einen Rest aus der Gruppe -O- oder -NH- steht,

wobei $R^2$ für

a) eine Verbindung der Formel III,

worin $R^5$ oder $R^6$ die in Anspruch 2 genannte Bedeutung haben,

b) eine Verbindung der Formel VI,

worin $R^5$ oder $R^6$ die in Anspruch 2 genannte Bedeutung haben,

c) eine Verbindung der Formel IX,

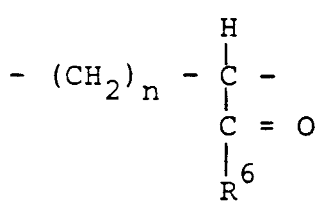

(IX)

wobei n für 3 oder 4 steht und $R^6$ die in Anspruch 2 genannte Bedeutung hat,

mit der Maßgabe, daß mehr als 5 Mol-% der Reste $R^1$ und/oder $R^2$ in dem Polykondensat der Formel I enthalten sind, wobei $R^1$ für eine Verbindung der Formel II oder $R^2$ für eine Verbindung der Formel III oder $R^1$ für eine Verbindung der Formel II und $R^2$ für eine Verbindung der Formel III steht,

oder daß mehr als 5 Mol-% der Reste $R^1$ und $R^2$ in dem Polykondensat der Formel I enthalten sind, wobei

a) $R^1$ für eine Verbindung der Formel V und $R^2$ für eine Verbindung der Formel VI steht, oder

b) $R^1$ für Phenyl[1,2], Phenyl[1,3] oder Phenyl[1,4] und $R^2$ für eine Verbindung der Formel VI steht.

**4.** Polykondensat, nach einem oder mehreren der Ansprüche 1 bis 3, dadurch gekennzeichnet, daß das Molekulargewicht im Bereich von 500 bis 1000000, bevorzugt 3000 bis 100000, liegt.

5. Verfahren zur Herstellung des Polykondensates nach einem oder mehreren der Ansprüche 1 bis 4, dadurch gekennzeichnet, daß man ein Dicarbonsäuredichlorid der Formel X,

ClOC - $R^1$ - COCl (X)

mit einem oder mehreren der Diole, Diamine, Dithioverbindungen oder Diazine der Formeln XI, XII, XIII oder XIV,

HO - $R^2$ - OH (XI)

$H_2N$ - $R^2$ - $NH_2$ (XII)

HS - $R^2$ - SH (XIII)

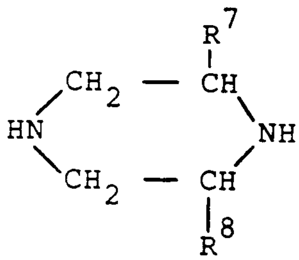

(XIV)

umsetzt, wobei $R^1$, $R^2$, $R^7$ und $R^8$ die in Anspruch 1 genannte Bedeutung haben.

6. Ultraschall-Kontrastmittel, bestehend aus Mikropartikeln, welche ein Gas und ein Polykondensat der Formel I nach einem oder mehreren der Ansprüche 1 bis 4 oder wie erhalten nach Anspruch 5 enthalten.

7. Verfahren zur Herstellung eines Ultraschall-Kontrastmittels nach Anspruch 6, dadurch gekennzeichnet, daß man
   a) eine Lösung eines oder mehrerer der Polykondensate der Formel I nach einem oder mehreren der Ansprüche 1 bis 4 oder wie erhalten nach Anspruch 5 oder eine Lösung dieses oder dieser Derivate, die zusätzlich ein oder mehrere weitere Polymere und/oder physiologisch unbedenkliche Hilfsstoffe enthält, sprühtrocknet.
   b) ein oder mehrere Polykondensate der Formel I nach einem oder mehreren der Ansprüche 1 bis 4 oderr wie erhalten nach Anspruch 5 in einem Lösungsmittel oder Lösungsmittelgemisch mit hohem Schmelzpunkt löst oder diese Polykondensate mit einem oder mehreren weiteren Polymeren und/oder physiologisch unbedenklichen Hilfsstoffen mischt und in einem Lösungsmittel oder Lösungsmittelgemisch mit hohem Schmelzpunkt löst und dann in ein kondensiertes kaltes Gas tropft und anschließend das Lösungsmittel entfernt, oder daß man
   c) ein oder mehrere Polykondensate der Formel I nach einem oder mehreren der Ansprüche 1 bis 4 oder wie erhalten nach Anspruch 5 in einem Lösungsmittel oder Lösungsmittelgemisch löst und anschließend gegebenenfalls nach Zusatz eines weiteren Lösungsmittels und/oder eines oder mehreren weiteren Polymeren ausfällt oder in Wasser dispergiert und die erhaltene Suspension von Lösungsmitteln befreit.

8. Verwendung der Ultraschall-Kontrastmittel nach Anspruch 6 zur Herstellung eines Diagnostikums oder Therapeutikums zur Untersuchung des Blutkreislaufes von Menschen oder Tieren oder zur Untersuchung von Hohlräumen in Menschen, Tieren oder Pflanzen.

9. Verwendung eines Polykondensats nach einem oder mehreren der Ansprüche 1 bis 4 oder wie erhalten nach Anspruch 5 zur Herstellung von Wirkstoffdepotzubereitungen mit kontrollierter Wirkstoffabgabe.

10. Diagnostikum oder Therapeutikum, enthaltend mindestens ein Ultraschall-Kontrastmittel nach Anspruch 6, neben physiologisch annehmbaren und pharmakologisch geeigneten Trägern und gegebenenfalls weitere Zusatz-und/oder Hilfsstoffen.

11. Verfahren zur Herstellung eines Diagnostikums oder Therapeutikums nach Anspruch 10, dadurch gekennzeichnet, daß man mindestens ein Ultraschall-Kontrastmittel nach Anspruch 6 mit einem physiologisch annehmbaren und pharmakologisch geeigneten Träger und gegebenenfalls weiteren Zusatz- und/oder Hilfsstoffen in eine geeignete Darreichungsform bringt.

**Patentansprüche für folgende Vertragsstaaten : ES, GR**

1. Verfahren zur Herstellung eines Polykondensats, das mindestens 95 Mol-% an wiederkehrenden Struktureinheiten der Formel I enthält,

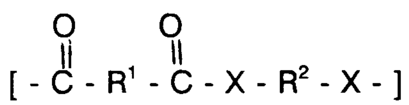

(I)

wobei $R^1$ für

a) Verbindung der Formel II

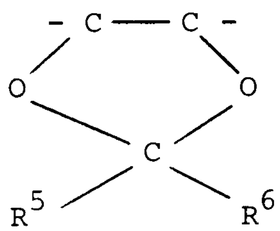

(II)

worin $R^5$ und $R^6$ inerte Reste bedeuten,

b) geradkettiges oder verzweigtes Alkyl, Alkenyl, Cycloalkyl oder Cycloalkenyl, die mit einem oder mehreren inerten Resten substituiert sein können,

c) ein- oder mehrkerniges Aryl, ein oder mehrfach substituiert durch inerte Reste,

d) Verbindung der Formel V,

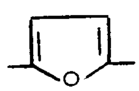

(V)

e) heterocyclischer Rest, der ein- oder mehrkernig und ein- oder mehrfach durch inerte Reste substituiert sein kann steht,

wobei X für

a) -O-,

b) -NH- oder

c) -S-

steht,

wobei $R^2$ für

a) Verbindung der Formel III,

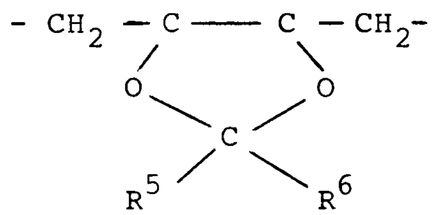

(III)

worin $R^5$ und $R^6$ die obengenannte Bedeutung haben,
b) geradkettiges oder verzweigtes Alkyl, Alkenyl, Cycloalkyl oder Cycloalkenyl, die mit einem oder mehreren inerten Resten substituiert sein können,
c) ein- oder mehrkerniges Aryl, ein- oder mehrfach substituiert durch inerte Reste,
d) heterocyclischer Rest, der ein- oder mehrkernig und ein- oder mehrfach durch inerte Reste substituiert sein kann, oder
e) Verbindung der Formel VI,

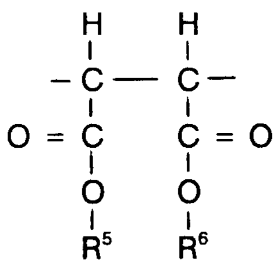

(VI)

worin $R^5$ und $R^6$ die obengenannte Bedeutung haben,
mit der Maßgabe, daß mehr als 5 Mol-% der Reste $R^1$ und/oder $R^2$ in dem Polykondensat der Formel I enthalten sind, wobei $R^1$ für eine Verbindung der Formel II oder $R^2$ für eine Verbindung der Formel III oder $R^1$ für eine Verbindung der Formel II und $R^2$ für eine Verbindung der Formel III steht,
oder daß mehr als 5 Mol-% der Reste $R^1$ und $R^2$ in dem Polykondensat der Formel I enthalten sind, wobei
a) $R^1$ für eine Verbindung der Formel V und $R^2$ für eine Verbindung der Formel VI steht, oder
b) $R^1$ für Phenyl[1,2], Phenyl[1,3] oder Phenyl[1,4] und
$R^2$ für eine Verbindung der Formel VI steht, dadurch gekennzeichnet, daß man ein Dicarbonsäuredichlorid der Formel X,

$ClOC - R^1 - COCl$ (X)

mit einem oder mehreren der Diole, Diamine, Dithioverbindungen oder Diazine der Formeln XI, XII, XIII oder XIV,

$HO - R^2 - OH$ (XI)

$H_2N - R^2 - NH_2$ (XII)

$HS - R^2 - SH$ (XIII)

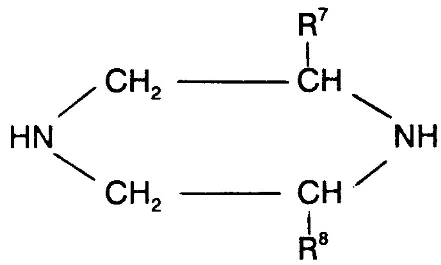

(XIV)

umsetzt, wobei $R^1$, $R^2$, $R^7$ und $R^8$ die oben genannte Bedeutung haben.

**2.** Verfahren nach Anspruch 1, wobei $R^1$ in der Formel I für
a) geradkettig oder verzweigtes ($C_1$-$C_{18}$)-Alkyl oder geradkettig oder verzweigtes ($C_1$-$C_{18}$)-Alkenyl, das eine oder mehrere Doppelbindungen enthält,
b) geradkettig oder verzweigtes ($C_1$-$C_{18}$)-Alkyl oder geradkettig oder verzweigtes ($C_1$-$C_{18}$)-Alkenyl, das eine oder mehrere Doppelbindungen enthält, ein- oder mehrfach substituiert durch
1) ($C_5$-$C_7$)-Cycloalkyl,
2) ($C_5$-$C_7$)-Cycloalkenyl, mit einer oder mehrerer Doppelbindungen,
3) Halogen, wie Fluor oder Chlor,
4) Mercapto,
5) Hydroxy,
6) ($C_1$-$C_6$)-Alkoxy,
7) Phenoxy,
8) Phenoxy, ein- oder mehrfach substituiert durch
8.1 Halogen, wie Fluor, Chlor, Brom oder Jod,
8.2 ($C_1$-$C_6$)-Alkyl,
8.3 ($C_1$-$C_6$)-Alkoxy,
8.4 Nitro oder
8.5 Carbethoxy,
9) Naphthyloxy,
10) Benzyloxy,
11) Benzyloxy, ein- oder mehrfach substituiert im Arylteil durch
11.1 Methoxy,
11.2 Carboxyamido,
11.3 Amino,
11.4 Halogen, wie Fluor, Chlor, Brom oder Jod,
11.5 Nitro oder
11.6 Methyl,
12) Amino,
13) Monoalkylamino, mit bis zu 7 C-Atomen,
14) Monoalkylamino, mit bis zu 7 C-Atomen, ein- oder mehrfach substituiert im Alkylteil durch
14.1 Hydroxy,
14.2 Carboxy,
14.3 Carboxyamid,
14.4 Carboethoxy,
14.5 Amino,
14.6 ($C_1$-$C_6$)-Alkylamino,
14.7 Di-($C_1$-$C_6$)-alkylamino,
14.8 Piperidin oder
14.9 Morpholin,
15) Dialkylamino, mit bis zu 7 C-Atomen, oder
16) Dialkylamino, mit bis zu 7 C-Atomen, substituiert wie in b) 14.1 bis 14.9 definiert,
c) Aryl, mit 5 bis 14 Kohlenstoffatomen, gegebenenfalls ein- oder zweifach substituiert durch
1.1 ($C_1$-$C_6$)-Alkyl,
1.2 ($C_2$-$C_6$)-Alkenyl,
1.3 ($C_1$-$C_6$)-Alkylcarboxy,

1.4 Hydroxy,
1.5 Halogen, wie Fluor, Chlor, Brom oder Jod,
1.6 Acetoxy,
1.7 ($C_1$-$C_6$)-Alkylcarboxyamid,
1.8 Sulfonamid,
1.9 Nitro,
1.10 ($C_1$-$C_6$)-Alkyloxy oder
1.11 Amino,

d) Verbindung der Formel IV,

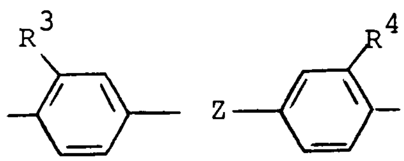

(IV)

worin $R^3$ oder $R^4$ unabhängig voneinander
1) Wasserstoff oder
2) inerter Rest bedeuten und

Z für eine Verbindung aus der Gruppe,
1) -O-,
2) -S-,
3)

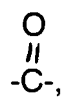

4) $-SO_2-$,
5)

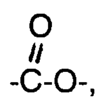

6) $-C_nH_{2n}-$, worin n eine ganze Zahl von 1 bis 10 bedeutet und die Kohlenstoffkette gerade oder verzweigt sein kann, oder
7) -O-Aryl-O, worin Aryl wie in c) definiert ist, steht,

e) Verbindung der Formel II,

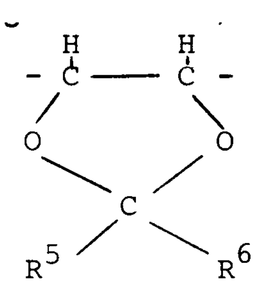

(II)

worin $R^5$ oder $R^6$ unabhängig voneinander
1) Wasserstoff,

2) Alkyl oder Alkenyl mit bis zu 18 C-Atomen,
3) Alkyl oder Alkenyl mit bis zu 18 C-Atomen, ein- oder mehrfach substituiert wie in b) 1 bis 16 definiert,
4) Aryl, mit 6 bis 14 Kohlenstoffatomen,
5) Aryl, mit 6 bis 14 Kohlenstoffatomen, ein- oder mehrfach substituiert wie in c) 1.1 bis 1.11 definiert, bedeuten oder
f) Verbindung der Formel V,

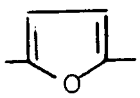

(V)

steht,
wobei X für
a) -O-,
b) -NH- oder
c) -S-
steht,

wobei $R^2$ für
a) Verbindung der Formel III,

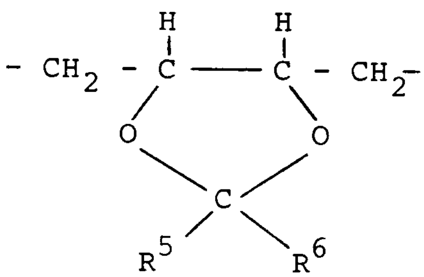

(III)

worin $R^5$ oder $R^6$ die obengenannten Bedeutungen haben,
b) Verbindung der Formel VI,

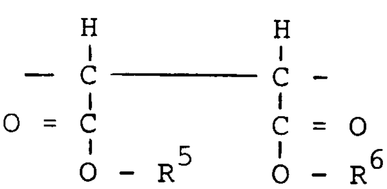

(VI)

worin $R^5$ oder $R^6$ die obengenannten Bedeutungen haben,
c) geradkettig oder verzweigtes Alkyl oder Alkenyl, mit 1 bis 18 C-Atomen,
d) geradkettig oder verzweigtes Alkyl oder Alkenyl, mit 1 bis 18 C-Atomen, ein- oder mehrfach substituiert durch einen Rest aus der Gruppe,
1) Carboxy,
2) Carboxy, worin die Hydroxygruppe ersetzt ist durch einen Rest aus der Gruppe,
2.1 -O- $(C_1-C_8)$-Alkyl,
2.2 Halogen, wie Fluor oder Chlor,
2.3 -O- $(C_1-C_4)$-Alkylcarbonyl,
2.4 $(C_1-C_6)$-Alkylamino,

2.5 ($C_1$-$C_6$)-Alkylamino, ein- oder mehrfach substituiert im Alkylteil durch einen Rest aus der Gruppe,
2.5.1 Hydroxy,
2.5.2 Mercapto,
2.5.3 -O- ($C_2$-$C_4$)-Alkyl,
2.6 genetisch kodierbare L-Aminosäure,
2.7 genetisch kodierbare L-Aminosäure, ein- oder mehrfach substituiert durch,
2.7.1 ($C_1$-$C_4$)-Alkyl,
3) Hydroxy,
4) Halogen, wie Fluor, Chlor, Brom oder Jod,
5) Amino,
6) Mercapto oder
7) Alkyl mit bis zu 8 C-Atomen,
e) Cyclische Verbindung der Formel VII,

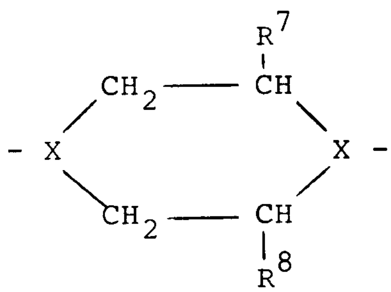

(VII)

worin $R^7$ oder $R^8$, unabhängig voneinander
1) Wasserstoff oder
2) ($C_1$-$C_3$)-Alkyl bedeuten
und X für N steht, oder
f) Aryl, mit 5 bis 14 Kohlenstoffatomen, gegebenenfalls ein- oder zweifach substituiert durch
1.1 ($C_1$-$C_6$)-Alkyl,
1.2 ($C_2$-$C_6$)-Alkenyl,
1.3 ($C_1$-$C_6$)-Alkylcarboxy,
1.4 Hydroxy,
1.5 Halogen, wie Fluor, Chlor, Brom oder Jod,
1.6 Acetoxy,
1.7 ($C_1$-$C_6$)-Alkylcarboxyamid,
1.8 Sulfonamid,
1.9 Nitro,
1.10 ($C_1$-$C_6$)-Alkyloxy oder
1.11 Amino,
mit der Maßgabe, daß mehr als 5 Mol-% der Reste $R^1$ und/oder $R^2$ in dem Polykondensat der Formel I enthalten sind, wobei $R^1$ für eine Verbindung der Formel II oder $R^2$ für eine Verbindung der Formel III oder $R^1$ für eine Verbindung der Formel II und $R^2$ für eine Verbindung der Formel III steht,
oder daß mehr als 5 Mol-% der Reste $R^1$ und $R^2$ in dem Polykondensat der Formel I enthalten sind, wobei
a) $R^1$ für eine Verbindung der Formel V und $R^2$ für eine Verbindung der Formel VI steht, oder
b) $R^1$ für Phenyl[1,2], Phenyl[1,3] oder Phenyl[1,4] und $R^2$ für eine Verbindung der Formel VI steht.

**3.** Verfahren nach Anspruch 1 oder 2, wobei $R^1$ in der Formel I für
a) eine Verbindung der Formel II,
worin $R^5$ oder $R^6$ unabhängig voneinander
1) Wasserstoff, mit der Maßgabe das $R^5$ und $R^6$ nicht gleichzeitig Wasserstoff bedeuten oder
2) geradkettiges oder verzweigtes Alkyl oder Alkylen mit bis zu 18 C-Atomen bedeuten,
b) Furo[2,5] oder
c) Phenyl[1,4], Phenyl[1,2], Phenyl[1,3], steht,
X für einen Rest aus der Gruppe -O- oder -NH- steht,

wobei $R^2$ für

a) eine Verbindung der Formel III,
worin $R^5$ oder $R^6$ die in Anspruch 2 genannte Bedeutung haben,

b) eine Verbindung der Formel VI,
worin $R^5$ oder $R^6$ die in Anspruch 2 genannte Bedeutung haben,

c) eine Verbindung der Formel IX,

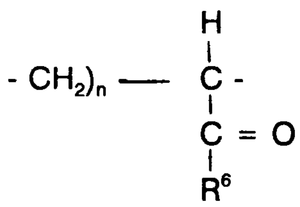

(IX)

wobei n für 3 oder 4 steht und $R^6$ die in Anspruch 2 genannte Bedeutung hat,

mit der Maßgabe, daß mehr als 5 Mol-% der Reste $R^1$ und/oder $R^2$ in dem Polykondensat der Formel I enthalten sind, wobei $R^1$ für eine Verbindung der Formel II oder $R^2$ für eine Verbindung der Formel III oder $R^1$ für eine Verbindung der Formel II und $R^2$ für eine Verbindung der Formel III steht,
oder daß mehr als 5 Mol-% der Reste $R^1$ und $R^2$ in dem Polykondensat der Formel I enthalten sind, wobei

a) $R^1$ für eine Verbindung der Formel V und $R^2$ für eine Verbindung der Formel VI steht, oder

b) $R^1$ für Phenyl[1,2], Phenyl[1,3] oder Phenyl[1,4] und $R^2$ für eine Verbindung der Formel VI steht.

**4.** Verfahren nach einem oder mehreren der Ansprüche 1 bis 3, dadurch gekennzeichnet, daß das Molekulargewicht des erhaltenen Polykondensats im Bereich von 500 bis 1000000, bevorzugt 3000 bis 100000, liegt.

**5.** Verfahren zur Herstellung eines Ultraschall-Kontrastmittels, bestehend aus Mikropartikeln, welche ein Gas und ein Polykondensat der Formel I erhalten nach einem oder mehreren der Ansprüche 1 bis 4 enthalten, dadurch gekennzeichnet, daß man

a) eine Lösung eines oder mehrerer der Polykondensate der Formel I erhalten nach einem oder mehreren der Ansprüche 1 bis 4 oder eine Lösung dieses oder dieser Derivate, die zusätzlich ein oder mehrere weitere Polymere und/oder physiologisch unbedenkliche Hilfsstoffe enthält, sprühtrocknet,

b) ein oder mehrere Polykondensate der Formel I erhalten nach einem oder mehreren der Ansprüche 1 bis 4 in einem Lösungsmittel oder Lösungsmittelgemisch mit hohem Schmelzpunkt löst oder diese Polykondensate mit einem oder mehreren weiteren Polymeren und/oder physiologisch unbedenklichen Hilfsstoffen mischt und in einem Lösungsmittel oder Lösungsmittelgemisch mit hohem Schmelzpunkt löst und dann in ein kondensiertes kaltes Gas tropft und anschließend das Lösungsmittel entfernt, oder daß man

c) ein oder mehrere Polykondensate der Formel I erhalten nach einem oder mehreren der Ansprüche 1 bis 4 in einem Lösungsmittel oder Lösungsmittelgemisch löst und anschließend gegebenenfalls nach Zusatz eines weiteren Lösungsmittels und/oder eines oder mehreren weiteren Polymeren ausfällt oder in Wasser dispergiert und die erhaltene Suspension von Lösungsmitteln befreit.

**6.** Verwendung der Ultraschall-Kontrastmittel nach Anspruch 5 zur Herstellung eines Diagnostikums oder Therapeutikums zur Untersuchung des Blutkreislaufes von Menschen oder Tieren oder zur Untersuchung von Hohlräumen in Menschen, Tieren oder Pflanzen.

**7.** Verwendung eines Polykondensats erhalten nach einem oder mehreren der Ansprüche 1 bis 4 zur Herstellung von Wirkstoffdepotzubereitungen mit kontrollierter Wirkstoffabgabe.

**8.** Verfahren zur Herstellung eines Diagnostikums oder Therapeutikums, dadurch gekennzeichnet, daß man mindestens ein Ultraschall-Kontrastmittel erhalten nach Anspruch 5 miteinem physiologisch

annehmbaren und pharmakologisch geeigneten Träger und gegebenenfalls weiteren Zusatz- und/oder Hilfsstoffen in eine geeignete Darreichungsform bringt.